Europäisches Patentamt

European Patent Office

Office européen des brevets

(11) Publication number: **0 294 132**
**A2**

# EUROPEAN PATENT APPLICATION

(21) Application number: 88304907.4

(22) Date of filing: 31.05.88

(51) Int. Cl.⁴: **C12N 9/64** , //**A61K37/54**

(30) Priority: 03.06.87 US 56927

(43) Date of publication of application:
07.12.88 Bulletin 88/49

(84) Designated Contracting States:
**AT BE CH DE ES FR GB GR IT LI LU NL SE**

(71) Applicant: SMITHKLINE BECKMAN
CORPORATION
One Franklin Plaza P O Box 7929
Philadelphia Pennsylvania 19103(US)

(72) Inventor: **Dephillips, Peter Arletti**
**577 Hidden Valley Road**
**King of Prussia Pennsylvania 19406(US)**
Inventor: **Franklin, Samuel Gregg**
**34 Sawgrass Lane**
**Newton Square Pennsylvania 19073(US)**
Inventor: **Johanson, Kyung Oh**
**28 Haymarket Lane**
**Bryn Mawr Pennsylvania 19010(US)**
Inventor: **McDevitt, Patrick Joseph**
**R.D. No. 2, Box 354**
**Douglassville Pennsylvania 19518(US)**
Inventor: **Sitrin, Robert David**
**237 Emerson Drive**
**Lafayette Hill Pennsylvania 19444(US)**
Inventor: **Strickler, James Edward**
**101 Pennsylvania Avenue**
**Havertown Pennsylvania 19083(US)**

(74) Representative: **Giddings, Peter John, Dr. et al**
**Smith Kline & French Laboratories Ltd.**
**Corporate Patents Mundells**
**Welwyn Garden City Hertfordshire AL7**
**1EY(GB)**

(54) Purification of tPA.

(57) A process for isolating tPA which comprises contacting a cell culture medium with a solid support onto which the tPA becomes adsorbed, washing the support with water buffered to < pH 5 and then eluting the tPA with an aqueous solution of an alkali or alkaline earth metal salt or a chaotrope buffered to < pH 5.

## Purification of tPA

### Field of the Invention

This invention relates to a process for purifying human tissue plasminogen activator from conditioned medium.

### Background of the Invention

Tissue Plasminogen Activator (tPA) is a large complex glycoprotein of approximately 70,000 molecular weight. Its structural features include 17 disulfide bridges which contribute to its complex conformation. Proper folding of the protein appears to be essential to its activity. For this reason, its seems, tPA produced by recombinant E. coli is largely inactive. Efforts at refolding E. coli-derived tPA have not been very successful because the refolding is difficult to direct and control, leading to incomplete and/or incorrect refolding.

Rijken et al., Biochem. Biophys. Acta 580:140 (1979), reports purification of tPA from uterine cell culture by (a) extraction at pH 4.2; (b) ammonium sulfate precipitation; (c) zinc chelate agarose chromatography (pH 7.5); (d) n-butyl agarose chromatography (pH 7.5), (e) Concanavalin A agarose chromatography (pH 7.5); and (f) gel filtration on Sephadex® G-150.

Meyhack et al., EP-A-143,081, at pages 19-20, suggest purification of tPA from yeast cell culture "by conventional means, such as salt precipitation, gel electrophoresis, dialysis, ion exchange chromatography, size exclusion chromatography, HPLC or reverce phase HPLC, molecular sizing on a suitable Sephadex® column, or the like." Specifically exemplified in Examples 20 and 21 are ammonium sulfate precipitation and affinity chromatography using anti-tPA antibodies and the Erythrina latissima trypsin inhibitor (DE-3).

Collen et al., EP-A-41,766, disclose purification of tPA from melanoma cell culture by (a) chromatography on zinc chelate agarose (pH 7.5), eluting with imidazole; (b) chromatography on lectin agarose (Concanavalin A Sepharose®) (pH 7.5), eluting with alpha-D-methylmannoside (0.3M) and thiocyanate (1M); and (c) gel filtration on cross-linked dextran (Sephadex® G-150).

Kruithof et al., Biochem. J. 226:631-636 (1985), disclose a purification scheme for tPA produced by serum-independent Bowes melanoma cells which includes the steps of (a) centrifuging conditioned medium; (b) adding Tween® 80 and $NaN_3$ to the supernatant and adjusting the pH to 4.5 with 6 M HCl; (c) passing the solution over a SP-Sephadex® column; and, (d) eluting the tPA with NaCl in a sodium acetate buffer at pH 4.5.

Dodd et al., FEBS 209(1):13 (1986) report purification of tPA by zinc chelate and lysine column chromatography.

Wallen et al., Eur. J. Biochem. 133: 681 (1983), report purification of tPA from melanoma cell culture by (a) affinity chromatography using anti-porcine tPA coupled to Sepharose® (pH 7.3), eluting with thicyanate (3M); (b) affinity chromatography on arginine-Sepharose® (pH 7.3), eluting with guanidinium chloride (1M); and (c) gel filtration on Sephadex® G-150.

Murakami et al., U.S. Patent 4,552,760, suggest purification of tPA by affinity chromatography using fibrin agarose (fibrin Sepharose®), lectin agarose, zinc chelate agarose or anti-tPA antibodies; ion exchange on a carboxymethyl agarose (CM Sepharose®); and gel filtration. A typical purication is stated to be: (a) ammonium sulfate precipitation; (b) ion exchange chromatography on CM cellulose: (c) affinity chromatography on lysine Sepharose®; and, (d) gel filtration (Sephacryl® S-200). As an example, Murakami et al. disclose purification from lung tissue cell culture by affinity chromatography using anti-urokinase antibodies and then on fibrin Sepharose®, followed by Concanavalin A Sepharose® and gel filtration (Sephadex® G-150).

Wilson, EP-A-113,319, report several tPA purification schemes at pages 15-17, including several of the above-mentioned procedures, as well as chromatography on Affi-Gel® Blue and arginobenzamidine Sepharose®. Particularly exemplified is affinity chromatography using DE-3 Sepharose®. See, also, Example 5.

Wei, et al., EP-A-178,105, disclose purification of tPA by (a) dextran-polyethylene glycol extraction; (b) affinity chromatography using a dye; (c) affinity chromatography using anti-tPA antibodies; and, (d) gel filtration.

Malefyt et al., European Patent Application No. 86305794.9, filed July 28, 1986, disclose a pharmaceutical formulation of tPA which comprises tPA in a buffered solution at pH 3.5-5.5.

Hasegawa et al., U.S. Patent 4,568,544, disclose purification of tPA from cell culture by a process which comprises ion exchange chromatography on CM Sepharose®, using a pH 8.9 buffer for elution.

Radcliffe et al., Arch. Biochem. Biophys. 189:185 (1978), describe purification of tPA from plasma by affinity chromatography on lysine coupled to Sepharose® 4B at pH 7.5, eluting with NaCl, followed by gel filtration on Sephadex® G-150 (pH 7.5).

## Summary of the Invention

This invention lies in the discovery of process steps, and of a preferred complete process, which can be used to purify tPA from cell culture. Specifically, in one aspect, this invention is a "capture step" for initially isolating tPA from conditioned medium. In this aspect, the invention comprises contacting the medium with a solid support which adsorbs the tPA; washing the support with water buffered to <pH 5; and, eluting the tPA with an aqueous solution of an alkali or alkaline earth metal salt or a chaotrope buffered to <pH 5.

In another aspect, the invention is a process for purifying tPA from an impure aqueous solution containing the tPH which process comprises contacting the solution with a solid hydrophobic support whereby tPA is adsorbed to the support and then eluting the adsorbed tPA from the support with a polar organic solvent.

In a further aspect, this invention is a process for purifying tPA from an impure solution containing the tPA which comprises flowing the solution through a size exclusion gel wherein the particle size is less than 50 um and has a separation range of 1000 to 300,000 molecular weight and collecting the tPA.

In another aspect, this invention is a process for purifying tPA from conditioned medium from a cell culture which produces tPA which comprises:

(i) contacting the conditioned medium with a solid support to which the tPA becomes adsorbed, washing the support with water buffered to <pH 5 and then eluting the tPA with an aqueous solution of an alkali or alkaline earth metal salt and/or a chaotrope buffered to <pH 5;

(ii) selectively precipitating the tPA from the eluant of step (i) and redissolving the precipitate in an aqueous solution containing a chaotrope at pH 2-5.5;

(iii) contacting the redissolved precipitate from step (ii) with an affinity support and eluting the tPA in an aqueous solution containing a chaotrope at <pH 5;

(iv) contacting the eluate of step (iii) with a hydrophobic support whereby the tPA is adsorbed to the support and then eluting the adsorbed tPA from the support with a polar organic solvent at pH 2-5.5;

(v) contacting the eluate of step (iv) with an ion exchange resin and collecting the tPA in an aqueous solution containing a chaotrope at <pH5;

(vi) passing the solution of tPA from step (v) through a size exclusion gel wherein the particle size is less than 50 um and the separation range of the gel is 1000 to 300,000 molecular weight and collecting the purified tPA.

## Detailed Description of the Invention

This invention comprises three process steps which can be used in a process for isolating and purifying tPA from an impure solution thereof. These are an "initial capture step," a reversed-phase chromatography procedure, and a size exclusion chromatography procedure. Each of these steps can be combined with other process steps including the other process steps of the invention to arrive at a complete process for isolating and purifying tPA from a producing recombinant or native, prokaryotic or eukaryotic cell culture.

These and other useful process steps are described hereinbelow in the context of an illustrative complete process of the invention which process comprises the "initial capture step," the reversed-phase chromatography procedure, an ion exchange chromatography procedure and the size exclusion chromatography procedure. In the description of this process below, between the "initial capture step" and the reversed-phase chromatography, a selective precipitation and a second adsorption chromatography step are included. This process of the invention is designed for isolation and recovery of bioactive tPA from

3

conditioned medium, that is, medium harvested from a cell culture producing tPA, such process is especially useful in the case of media which contain serum or serum components especially albumins. Thus, such process can be used to purify tPA expressed by recombinant or other cell culture, including recombinant Chinese hamster ovary (CHO) recombinant hamster kidney cells, recombinant mouse or rat myeloma cells, melanoma cell culture and recombinant insect cell culture.

Conditioned medium is harvested by decanting from a batch culture system or by collecting medium from a flow-through system. The medium is optionally clarified such as by filtration and/or centrifugation, the tPA is initially isolated by adsorption chromatography. This step is referred to as the "initial capture step" because it results in isolation of most of the tPA while substantially reducing the volume of medium for further processing. Such adsorption chromatography employs a solid support capable of handling a large volume of medium at a high flow rate and with high efficiency. An example of such adsorption chromatography is chromatography using an affinity ligand such as polyclonal or monoclonal antibodies, a metal chelating agent, lysine, arginine, fibrin, DE-3, various dyes, boronic acid, phenylboronate, concanvaline A, p-aminobenzamidine and benzamidine. The preferred capture step is metal chelate chromatography, especially zinc chelate chromatography. Such technique is generally described by Porath et al., Nature 258:598 (1975). The support can be, e.g., a soft gel such as dextran, agarose or polyacrylamide gel, a rigid gel such as Fractogel® vinyl polymer gel, 32-63 um size (Pierce Chemical Co., Rockford, Illinois) or any other support capable of adsorbing tPA which is staple below pH 5.

It has now been discovered that the capture step can be enhanced, and indeed can contribute significantly to purification, by washing the adsorption support with water buffered to below pH 5 prior to eluting the tPA and by eluting tPA at below pH 5 in an aqueous solution of an alkali or alkaline earth metal salt, e.g., NaCl, KCl, MgCl$_2$ or CaCl$_2$, or a chaotrope, e.g., guanidine, guanidinium chloride, thicyanate, urea or ethylene glycol.

It has been found, for example, that although washing a zinc chelate column at about neutral pH in accordance with the standard procedure does remove a significant amount of contaminants from conditioned medium, a second wash at <pH 5 unexpectedly removes a further significant amount of contaminants. For example, in a representative experiment, in which a zinc chelate column was washed completely first with water buffered to pH 6 with ammonium acetate and then with water buffered to pH 4.5 with ammonium acetate prior to elution with 0.25 M NaCl buffered to pH 4.5 with ammonium acetate, the first wash removed an amount of contaminants approximately equal to the amount of tPA recovered and the second wash removed an unexpectedly large amount of contaminants, equal to about 10% of the amount of tPA as determined by comparison of peaks in a chromatogram showing adsorbance at 280 nm.

In carrying out the capture step, washing is preferably carried out initially at greater than pH 5, e.g., pH 6-8 and preferably about pH 6, after which the pH is lowered to <pH 5, e.g., pH 4.0-4.9, preferably about pH 4.5. The buffering agent can be any water-soluble acid/salt, for example, acetic acid, adipic acid, citric acid, lactic acid, tartaric acid, aspartic acid and glutamic acid, and their salts. The preferred buffer is ammonium acetate.

To elute the tPA, a solution of an inorganic alkali or alkaline earth metal salt or of a chaotrope at <pH 5, e.g., pH 4.0-4.9, is used. The preferred elution solution is 0.1 to 1.0 M, preferably 0.25 M, sodium chloride in 0.1 M ammonium acetate (pH 4.5).

The impure solution obtained from the capture step is preferably further partially purified prior to reversed-phase chromatography. The partial purification can be accomplished by any means including, for example, by selective precipitation, ion exchange, affinity chromatography, filtration, normal phase chromatography and size exclusion, or any combination of these or other steps. Preferably, the impure solution has been partially purified such that the tPA is at least about 50% pure and preferably about 75 to 95% pure, based on total protein content prior to the reversed-phase procedure.

Illustrative of process steps for achieving a desired degree or purity prior to the reversed-phase procedure are selective precipitation, e.g., salt precipitation, or other selective precipitation procedure, and at least one adsorption step, e.g., affinity chromatography or immunoaffinity chromatography.

Salting out is carried out by adding, for example, sulfate, phosphate or citrate salts with monovalent captions such as ammonium, potassium or sodium in a step-wise gradient. Fractions containing tPA predipitate, after washing, are resuspended at low pH, e.g., pH 2 - 5.5. Other agents for selective precipitation include organic solvents such as C 1-3 alcohol, acetone, acetonitrile or tetrahydrofuran; organic polymers such as polyethylene glycol; polyelectrolytes such as polyacrylates; caprylic acid salts; and, rivanol.

For the second adsorption chromatography step, a variety of ligands can be employed. These include the affinity ligands mentioned above. Elution can be specific, i.e., affinity elution, or general, such as by altering the pH or ionic strength or otherwise altering the conformation of the tPA.

It has been found to be useful, for example to employ a benzamidine support, e.g., benzamidine-agarose. For such procedure, the ammonium sulfate pellet is preferably resolubilized in a buffered solution at about pH 5, e.g., 0.1 M ammonium acetate, in a presence of a chaotraope and of a detergent, e.g., 0.01% Tween-80. The solution is preferably diluted to an absorbance at 280 nm of about 5 to 6 units and applied to the support in the amount of 8 to 15 $A_{280}$ units, preferably about 12 $A_{280}$ units, per mL of gel. Preferably, the concentration of the chaotrope is low, e.g., up to 4 M guanidine or guanidinium chloride, up to 50% (v/v) ethylene gylcol, up to 6 M urea or up to 2 M potassium thiocyanate. The most preferred chaotrope is about 0.1 to 0.4 M guanidinum chloride. Elution is accomplished by lowering the pH to <pH 5, e.g., about pH 4.

In the preferred process of the invention, the partially purified solution from the initial capture step which has been treated by selective precipitation and by a second adsorption chromatography step is then treated by reversed-phase chromatography.

Reversed-phase chromatography utilizes a hydophobic support to adsorb a protein from an aqueous solvent, followed by selective elution with a polar, organic solvent, typically an alcohol-water mixture. The polar organic solvent causes elution by displacing hydrophobic domains on the polypeptide. Many proteins cannot be purified in this way due to the use of harsh conditions which can cause a protein, especially a protein for which the activity is conformation dependent, to lose activity.

The reversed-phase chromatography is carried out, typically, using an alkylsilica support, although any hydrophobic support can be employed, e.g., alkyl substituted carbohydrate or alumina gels. The preferred support is a $C_2$ to $C_{18}$ bonded silica, preferably $C_4$. Other silanes include octyldimethyl, octadecyldimethyl, phenyldimethyl, actyl, octadecyl and phenyl silanes. Particle size can be 5-100 um, preferably about 15-20 um. Typical pore size is 200 to 500 A.

The impure solution prior to the reversed-phase step preferably is adjusted to a very low pH, e.g., 1.5 to 3.5, with, e.g., heptafluorobutyric acid, acetic acid, phosphoric acid or trifluoroacetic acid. Inclusion of a chaotrope or an organic solvent in the impure solution, e.g., 0.1 to 0.4 M guanidinium chloride, improves separating, apparently by disassociating tPA aggregates. Elution is typically accomplished by adding increasing amounts of an organic solvent to diminish hydrophobic interactions, e.g., acetonitrile, dioxane, tetrahydrofuran or a water-miscible alcohol, e.g., ethanol, methanol, n-propanol or isopropanol. The preferred elution gradient is 0.30% isopropanol containing 0.1% trifluracetic acid.

Following the reversed-phase chromatography, one or more final cleanup steps are desirable to produce a product which is pharmaceutical grade tPA. Preferably, these are ion exchange chromatography using standard ion exchange resins or functional supports with elution at low pH, i.e., pH 2 to 5.5, in a presence of a chaotrope, followed by size exclusion.

The ion exchange step serves to concentrate the tPA and to remove the organic solvent. Preferably, this procedure is carried out using a cation exchange support, such as a carboxymethyl or sulfopropyl support. Alternatively, an anion exchange support such as a diethylaminoethyl or quarternary aminoethyl support can be used. Fractions collected from the ion exchange column which contains tPA can then be treated by size exclusion.

The size exclusion step is carried out using a small particle packing, e.g., less than about 50 um, having a separation range of 1000 to 300,000 molecular weight. It has been discovered that by use of such gel, residual traces of high molecular weight contaminants can be removed. For example, Superose® 12 cross-linked agarose (Pharmacia, Piscataway, New Jersey), which has an average particle size of 20-40 um and a separation range of 1,000 to 300,000 molecular weight, has been discovered to readily separate tPA, which has a molecular weight of about 70,000, from a high molecular weight (< about 90,000 MW) contaminant encountered in tPA preparation from conditioned medium containing serum after carrying out the above-described procedures prior to size exclusion. Such separation of physically similar proteins by size exclusion chromatography was unexpected. This procedure also removes residual tPA aggregates, including dimers, and nucleic acids and, if present, virus particles.

The size exclusion step is preferably carried out in a presence of chaotrope, preferably 2 M guanidinium chloride, adjusted to <pH 5. The chaotrope disrupts aggregates and permits the efficient separation realized in this step. Conveniently, the tPA can be eluted from the cation exchange resin with an elution buffer having such chaotrope. This will enhance disassociation and minimize formation of tPA aggregates.

Fractions from the size exclusion step which contain tPA are collected from final formulation, sterile filtration and packaging. Prior to final formulation, salt can be removed by, e.g., dialysis, diafiltration, evaporation or buffer exchange on, for example, Sephadex® G-25, Biogel® P2 or Biogel® P4.

Each process step of the invention, as noted previously, can be combined with other process steps which may or may not be process steps of the invention. Preferably, the process steps of the invention are combined as illustrated in Scheme I, which follows.

## Scheme I

CONDITIONED MEDIUM
    ↓ Zn Chelate Fractogel®

ELUATE
    ↓ Ammonium Sulfate

PRECIPITATE
    ↓ Wash/Dissolve/Filter

FILTRATE
    ↓ Benzamidine Agarose

ELUATE
    ↓ HPLC (reversed-phase)

FRACTIONS
    ↓ CM-Sepharose®

ELUATE
    ↓ Superose® 12

FRACTIONS
    ↓ Buffer Exchange/Diafiltration
      Concentration

CONCENTRATE
    ↓ 0.2 µm Filter

BULK tPA PRODUCT

The process illustrated in Scheme 1 employs chromatographic columns suitable for large scale processing. Use of low pH throughout the entire procedure is advantageous due to the increased stability and solubility of tPA and, unexpectedly, the low pH permits more effective separation. Also, low pH tends to suppress proteolytic enzymes in conditioned medium. Also, conditions of the process are such as to inactivate possible viral contaminants. Most importantly, a high yield of non-denatured, fully active, pure tPA is obtained through use of this process.

Following the purification, the pure tPA solution can be sterile filtered and formulated for subsequent pharmaceutical administration, such as described by Malefyt et al., European Patent Application No. 86305794.9.

## Example

The example which follows is illustrative of the preferred embodiment of the invention, but is not limiting. The process is summarized in Scheme 1 above. Conditioned medium was harvested from recombinant Chinese hamster ovary (CHO) cells grown in a harvest medium of Minimal Essential Medium (MEM) Alpha (+) with nucleosides (Hazelton Research Products, Denver, Pennsylvania) supplemented with fetal calf serum (5% V/V), gentamicin (.05 g/L), methotrexate (200 nM), dexamethasone (1 uM) and NaHCO$_3$ (2.2 g/L).

## 1 Zinc Chelate Column

A column of zinc chelate Fractogel (1.5 to 7 L) is prepared within one day of use by equilibration of a column of iminodiacetic acid immobilized on Fractogel TSK HW-65F with five column volumes of 0.05 M EDTA, five column volumes of 0.05 M ammonium bicarbonate, five column volumes of deionized water, five column volumes of 7.3 mM ZnCl$_2$), and ten column volumes of Equilibration Buffer (1 M NaCl, 20 mM Tris-HCl) until no zinc ion can be detected in the effluent. Conditioned medium (100 to 500 L) is pumped onto the column at a flow rate of 6 to 50 L/hr, depending on column size. The column is washed with five column volumes of the Equilibration Buffer, 5 column volumes of 0.1 M ammonium acetate (pH 6.0) and 2 column volumes of 0.1 M ammonium acetate (pH 4.5). The product is then eluted with 5 column volumes of the Elution Buffer (0.1 M ammonium acetate, 0.25 M NaCl, pH 4.5). Fractions containing tPA as determined by the S-2251 assay (Weinberg et al., J. Immunol. Meth. 75: 289 (1984)) are pooled for further processing.

## 2 Ammonium Sulfate Precipation

Granular ammonium sulfate is added gradually to the solution of pooled fractions from the zinc chelate column to a final concentration of 52% of saturation. The suspension is stirred for 30 minutes of 4°C and allowed to stand for at least 12 hours at 4°C. The slurry is centrifuged in a Beckman J6B centrifuge (Beckman Instruments, Fullerton, California) at 4000 rpm for 30 minutes at 4°C and the pellet is collected and stored at -70°C until used in the next step.

Thawed ammonium sulfate pellets (200-300 g), containing an estimated 12 to 16 g of tPA as determined by the S-2251 assay, are resuspended in 2-3 L of 52% saturated ammonium sulfate and the slurry is centrifuged at about 9,000 x g in a Beckman JA10 rotor for 30 minutes at 4°C to yield a washed pellet for further processing.

## 3 Benzamidine Agarose Column

The washed ammonium sulfate pellet produced in the previous step is dissolved in 0.1 M ammonium acetate containing 0.2 M guanidinium chloride and 0.01% Tween-80 (pH 5.0) and the solution is diluted to an OD$_{280}$ of 5 to 6 (final volume approximately 7 L). The solution is filtered through an 0.22 micrometer filter and loaded onto a column packed with 2 L of benzamidine agarose [(immobilized p-aminobenzamidine coupled to cross-linked 4% beaded agarose via 6-aminocaproic acid (Pierce Chemical Company, Rockford, Illinois)] at a flow rate of 3 L/hr. The column is washed with a 8 L of a solution containing 0.1 M ammonium acetate, 0.3 M guanidinium chloride, 0.3 M potassium thiocyanate, 0.01% Tween-80, pH 5.0) at a flow rate of 8 L/hr. tPA is eluted from the column with 0.1 M ammonium acetate containing 0.2 M guanidinium chloride and 0.01% Tween-80 (pH 4.0) at a flow rate of 8 L/hr. Fractions containing tPA as determined by monitoring the UV absorbance of the effluent at 280 nm are pooled to a volume of approximately 5 L.

## 4 Reversed-phase Chromatography

The solution of pooled fractions from the benzamidine agarose column is adjusted at 4°C to a pH of 2.1 +/- 0.1 with trifluoroacetic acid (TFA) and chromatographed in approximately one gram portions on Vydac 214 TP, 300 Angstrom C-4 packing (15-20 micrometer silica) (Separations Group, Hesperia, California) in a 5.1 x 25 cm column, previously equilibrated with 0.1% aqueous trifluoroacetic acid, using a gradient of 0 to 30% isopropanol containing 0.1% trifluoroacetic acid at a flow rate of 80 mL/min. The desired product elutes at approximately 27% isopropanol as detected by monitoring the UV absorbance of the effluent at 220 nm.

## 5 CM Sepharose Chromatography

The solution of combines fractions from the reversed-phase chromatography contain tPA is diluted with an equal volume of 1 M ammonium acetate (pH 4.5) and pumped into a column of 400 mL of CM Sepharose (Pharmacia, Piscataway, New Jersey) at a flow rate of 100 mL/min. The column is washed with approximately 1.2 L of 0.5 M ammonium acetate (pH 4.5), 1.2 L of 1 M ammonium acetate (pH 4) and the desired product is eluted with 0.1 M ammonium acetate containing 2 M guanidinium chloride (pH 4.0). tPA in the effluent is detected by monitoring the UV adsorption at 280 nm. Fractions containing tPA (in approximately 600 to 800 mL) are pooled for further processing.

## 6 Superose-12 Chromatography

The product isolated from the CM Sepharose column is chromatographed in three to four 200-300 mL portions on an 8 L column of Superose 12 Prep Grade using 0.1 M ammonium acetate containing 2 M guanidinium chloride (pH 4.0). Fractions are pooled on the basis of purity as determined by HPLC analysis.

## 7 Buffer Exchange

The pool of fractions from the Superose-12 column containing the desired product is chromatography in two to three portions on an 8 L column of Sephadex G-25 using 0.1 M ammonium acetate (pH 4) to effect buffer exchange. The effluent is monitored by UV at 280 nm and fractions containing the desired product are pooled and concentrated in an ultrafiltration stirred cell equipped with PM-30 membrane to an $OD_{280}$ +/- 1. The concentrated solution is filtered through a 0.22 micrometer cartridge to yield a solution of pure bulk tPA for formulation.

Alternatively, the combined Superose-12 product is concentrated in an ultrafiltration-stirred cell equipped with a PM-30 membrane to an $OD_{280}$ of 22 +/- 1 and diafiltered against 10 volumes of 0.1 M ammonium acetate 0.22 micrometer cartridge to yield a solution of pure bulk tPA for formulation.

Table 1 describes the results of a typical production run starting from 1667 L of conditioned medium containing an estimated 20 g of tPA as determined by the S-2251 assay using an American Diagnostica activity standard (American Diagnostica, Greenwich, Connecticut) and assuming 700,000 IU/mg.

The first column in Table 1 lists the total amount of bioactivity in billions of international units (BIU) present at each step of the process as measured by the S-2251 assay using the American Diagnostica activity standard. The second column in Table 1 lists the cumulative percent recovery of biactivity during the course of the purification. The overall recovery of bioactivity was 37%. The third column lists the percent recovery per step.

Mass recovery of tPA during the process was determined by HPLC analysis. Levels of tPA expressed in grams present at each stage for steps after the zinc chelate capture column are listed in column 4. The amount of tPA present in conditioned medium cannot be determined by this assay. Column 5 lists the percent recovery of tPA per step for those steps assayed by HPLC analysis.

Column 6 lists the total protein content at each stage of the purification process. The total protein level (tPA plus all others) in conditioned medium containing 5% fetal calf serum (FCS) can be calculated to be 2 g/L since FCS contains 40 mg/mL protein. The bulk of the protein content of conditioned medium comes from the added FCS. The total amount of all proteins present (tPA plus contaminants) at each of the purification steps was estimated by UV at 280 nm assuming 1.8 $A_{280}$ units corresponds to 1 mg/mL protein.

The specific activity at each step of the process was calculated by dividing the S-2251 activity in IU (column 1) by the total protein content (Column 6) and is expressed in IU/mg.

## Table I

### Recovery and Purification of tPA for a Typical Batch

| SAMPLE | tPA | | | | | TOTAL PROTEIN[3] (G) | SPECIFIC ACTIVITY[4] (IU/MG) |
|---|---|---|---|---|---|---|---|
| | S-2251 ACTIVITY[1] | | | MASS BY HPLC | | | |
| | BIU[2] | PERCENT RECOVERY | PERCENT RECOVERY PER STEP | G | PERCENT RECOVERY PER STEP | | |
| Conditioned Medium (1667L) | 13.2 | 100 | - | - | - | 3300 | 4,000 |
| Zinc Chelate | 9.6 | 73 | 73 | - | - | - | - |
| Am. Sulfate Pellet | 10.3 | 78 | 107 | 14.4 | - | 20.0 | 515,000 |
| Benzamidine Agarose | 10.0 | 76 | 97 | 11.9 | 82 | 17.4 | 575,000 |
| Reversed Phase HPLC | 6.6 | 50 | 66 | 11.3 | 95 | 10.4 | 635,000 |
| CM Sepharose | 6.6 | 50 | 100 | 10.4 | 92 | 9.9 | 667,000 |
| Superose-12 | 6.6 | 50 | 100 | 8.5 | 81 | 8.0 | 825,000 |
| Bulk Product | 4.9 | 37 | 74 | 7.5 | 89 | 7.1 | 690,000 |

1. American Diagnostica Activity Standard

2. BIU = $10^9$ IU

3. Assuming 1.8 OD = 1 mg/ml Total Protein except for Conditioned Medium (5% serum) which is calculated to be 2 g/L Total Protein

4. Specific activity was calculated by dividing the total S-2251 activity (column 1) by the total protein content (column 6) at each step

Because of the technical difficulty of measuring trace qualities of nucleic acid contaminants, removal of DNA was determined by clearance factors using 32P-labelled DNA of about 500 base pairs for each step. The estimated aggregate clearance factor for the entire process is 6 x 10".

Virus inactivation by the above process is demonstrated by incubation of retrovirus for times and under conditions substantially identical to those of each step of the process. The aggregate inactivation factor for the reversed-phase and ion exchange steps is estimated to be in excess of 3.7 x 10".

The above description and examples fully disclose the invention and its preferred embodiments. The invention, however, is not limited to the precise constructions described herein but, rather, encompasses the full scope, including modifications of the claims which follow.

## Claims

1. A process for isolating tPA from conditioned medium from a cell culture which produces tPA which comprises contacting the medium with a solid support to which the tPA becomes adsorbed, washing the support with water buffered to <pH 5 and then eluting the tPA with an aqueous solution of an alkali or alkaline earth metal salt or a chaotrope buffered to <pH 5.

2. The process of claim 1 in which the washing step is initially carried out at >pH 5 after which the pH is lowered to pH 4.0-4.9.

3. The process of claim 1 in which the support contains a metal chelating ligand.

4. The process of claim 3 in which the support is a zinc chelate column and in which the support is washed at pH 4.0-4.9.

5. The process of claim 4 in which the tPA is eluted with 0.1 to 1.0 M NaCl.

6. The process of claim 5 in which the washing step is initially carried out at about pH 6 in 0.1 M ammonium acetate after which the pH is lowered to about pH 4.5 and in which the tPA is eluted with 0.24 M NaCl in 0.1 M ammonium acetate at pH 4.5.

7. A process for purifying tPA from an impure, aqueous solution containing the tPA which process comprises contacting the solution with a hydrophobic support whereby the tPA is adsorbed to the support and then eluting the adsorbed tPA from the support with a polar, organic solvent at pH 2-5.5.

8. The process of claim 7 wherein the impure solution is at least about 50% pure prior to contacting the solution with the hydrophobic support and wherein the hydrophobic support is a $C_{2-18}$ alkylsilica.

9. The process of claim 8 wherein the impure solution is adjusted to pH 1.5 to 3.5 and guanidine, guanidinium chloride, urea, thicyanate or ethylene glycol is added to the impure solution, prior to contacting the solution with the hydrophobic support.

10. The process of claim 8 wherein the impure solution is derived from conditioned medium from a cell culture which produces tPA by at least one adsorption chromatography step.

11. The process of claim 10 wherein the conditioned medium is partially purified by (i) adsorption chromatography using a zinc chelate support and eluting with salt at below pH 5; (ii) ammonium sulfate precipitation; and then (iii) adsorption chromatography, prior to contacting the solution with the hydrophobic support.

12. The process of claim 11 wherein the second adsorption chromatography is carried out using a benzamidine support and by eluting at <pH 5.

13. The process of claim 11 wherein following the elution of the adsorbed tPA from the hydrophobic support, the purified solution is contacted with an ion exchange resin and reluted at pH 2 to 5.5 in a presence of guanidine, guanidinium chloride, urea, thiocyanate or ethylene glycol.

14. A process for purifying tPA from an impure solution containing the tPA which comprises passing the solution through a size exclusion gel wherein the particle size is less than 50 um and the separation range of the gel is 1000 to 300,000 molecular weight and collecting the tPA.

15. The process of claim 14 wherein the solution contains guanidine, guanidinium chloride, urea, thiocyanate or ethylene glycol and is adjusted to <pH 5 prior to size exclusion.

16. The process of claim 15 wherein the size exclusion gel is Superose® 12.

17. The process of claim 16 wherein the solution contains 0.1 M ammonium acetate and 2 M guanidinium chloride and is about pH 4 prior to size exclusion.

18. A process for purifying tPA from conditioned medium from a cell culture which produces tPA which comprises the steps of:

(i) contacting the conditioned medium with a solid support to which the tPA becomes adsorbed, washing the support with water buffered to <pH 5 and then eluting the tPA with an aqueous solutiong of an alkali or alkaline earth metal salt and/or a chaotrope buffered to <pH 5;

(ii) selectively precipitating the tPA from the eluate of step (i) and redissolving the precipitate in an aqueous solution containing a chaotrope, quanidinium chloride, urea, thiocyanate or ethylene glycol at pH 2-5.5;

(iii) contacting the redissolved precipitate from step (ii) with an adsorption support and eluting the tPA in an aqueous solution containing a chaotrope at <pH 5;

(iv) contacting the eluate of step (iii) with a hydrophobic support whereby the tPA is adsorbed to the support and then eluting the adsorbed tPA from the support with a polar organic solvent at pH 2-5.5;

(v) contacting the eluate of step (iv) with an ion exchange resin and collecting the tPA in an aqueous solution containing a chaotrope at <pH 5;

(vi) passing the solution of tPA from step (v) through a size exclusion gel wherein the particle size is less than 50 um and the separation range of the gel is 1000 to 300,000 molecular weight and collecting the purified tPA.